(19) Europäisches Patentamt
European Patent Office
Office européen des brevets

(11) EP 0 711 542 B1

(12) EUROPEAN PATENT SPECIFICATION

(45) Date of publication and mention of the grant of the patent:
**18.07.2001 Bulletin 2001/29**

(51) Int Cl.$^7$: **A61K 7/13**, A61K 7/50

(21) Application number: **95116559.6**

(22) Date of filing: **20.10.1995**

(54) **Coloring shampoo composition**

Haartönungsshampoo

Composition de shampooing pour la coloration des cheveux

(84) Designated Contracting States:
**DE FR GB**

(30) Priority: **20.10.1994 JP 25504494**

(43) Date of publication of application:
**15.05.1996 Bulletin 1996/20**

(73) Proprietor: **KAO CORPORATION**
**Chuo-ku, Tokyo (JP)**

(72) Inventors:
- **Sakuma, Risa, c/o Kao Corporation Sumida-ku, Tokyo (JP)**
- **Yahagi, Kazuyuki, c/o Kao Corporation Sumida-ku, Tokyo (JP)**

(74) Representative:
**Hansen, Bernd, Dr. Dipl.-Chem. et al**
**Hoffmann Eitle,**
**Patent- und Rechtsanwälte,**
**Arabellastrasse 4**
**81925 München (DE)**

(56) References cited:
**EP-A- 0 137 178**
**EP-A- 0 367 926**
**EP-A- 0 531 943**
**BE-A- 641 262**
**DE-A- 1 492 196**
**FR-A- 1 472 976**
**GB-A- 986 712**
**GB-A- 2 142 348**
**US-A- 2 763 269**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

This invention relates to a coloring shampoo composition which is excellent in the hair-dyeing effect and detergency, causes little irritation and imparts a good touch to the hair while giving no squeak feel during shampooing.

In recent years, various coloring shampoos have been put on the market in order to satisfy the consumers' demand for changing the shade of the hair color. Among these products, those containing direct dyes and surfactants are exemplified by the compositions described in European Patent No. 137178A, European Patent No. 367926A and European Patent No. 46543A. However, each of these coloring shampoos has only a poor hair-dyeing power. Thus it is often observed that the hair dyed therewith suffers from fading or discoloration after shampooing several times.

Accordingly, there have been proposed coloring shampoos containing anionic surfactants, amine oxide-type surfactants and alkyl polyglucosides to make up for the limited hair-dyeing effects of the conventional coloring shampoos (JP-A-5-194159; the term "JP-A" as used herein means an "unexamined published Japanese patent application"). However it has been required by the consumers to develop coloring shampoos with furhter improved dyeing characteristics.

It is assumed that the dyeing characteristics can be improved by using a basic dye, which is a cationic direct dye, to thereby promote the adsorption of the dye on the hair. However, there arises a problem that when such a basic dye is used in a shampoo containing an anionic surfactant as the main base, the basic dye is hardly adsorbed on the hair. When a basic dye is used in a shampoo containing an alkyl glucoside which is a nonionic surfactant as the main base, the obtained product shows good dyeing characteristics but an insufficient detergency. In this case, furthermore, there arises a problem of a squeak feel of the hair during shampooing, when the basic dye is used in a large amount.

BE-A-641262 reveals a coloring composition for providing a semi-permanent color to the hair, comprising an aqueous medium in which a water-insoluble colorant is dispersed, wherein the dispersant is a combination of a fatty alcohol polyoxyethylene ether, urea and fatty alcohol. The colorant is used in an amount of 0.25 to 2% by weight. The ether is used in an amount of 0.5 to 5% by weight. According to the examples the amount thereof is 0.5 to 2% by weight.

Thus, an object of the present invention is to provide a coloring shampoo composition which has excellent dyeing characteristics, a long-lasting effect and a high detergency, causes little irritation and imparts a good touch to the hair without giving any squeak feel during shampooing.

As a result, they have successfully found out that a coloring shampoo composition, which exhibits an intense and long-lasting dyeing effect, has a high detergency and imparts a good touch to the hair while giving no squeak feel during shampooing, can be obtained by using a combination of a basic dye with a specific polyoxyethylene alkyl ether-type nonionic surfactant.

Accordingly, the present invention provides a coloring shampoo composition which comprises the components (A) and (B) as defined below:

(A) 7 to 50 % by weight of a polyoxyethylene alkyl ether-type nonionic surfactant represented by the following formula (1):

$$R^1O\text{-}(CH_2CH_2O)n\text{-}H \qquad (1)$$

wherein $R^1$ represents a linear or branched alkyl or alkenyl group having 10 to 20 carbon atoms; and n is a number of from 11 to 30; and

(B) 0.01 to 10 % by weight of a basic dye.

The coloring shampoo composition of the present invention may further contain :

(C) 0.1 to 30 % by weight of at least one nonionic surfactant selected from among those represented by the following formulae (2) to (6):

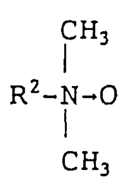

(2)

wherein $R^2$ represents a linear or branched alkyl or alkenyl group having 8 to 18 carbon atoms;

$$R^3\text{-}(OR^4)s\text{-}Gt \quad (3)$$

wherein $R^3$ represents a linear or branched alkyl or alkenyl group having 6 to 18 carbon atoms; $R^4$ represents an alkylene group having 2 to 4 carbon atoms; G represents a group originating in a reducing sugar having 5 or 6 carbon atoms; s is a number of from 0 to 10; and t is a number of from 1 to 10;

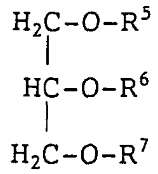

(4)

wherein either one of $R^5$, $R^6$ and $R^7$ represents $O{=}CR^8$ (wherein $R^8$ represents a linear or branched alkyl or alkenyl group having 6 to 18 carbon atoms), while others represent each a hydrogen atom;

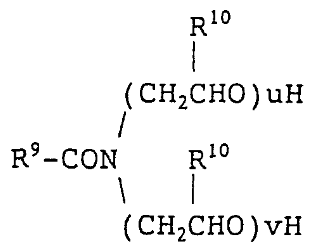

(5)

wherein $R^9$ represents a linear or branched alkyl or alkenyl group having 8 to 20 carbon atoms; $R^{10}$ is a hydrogen atom or methyl group; and u and v are each an integer of 0 to 10, provided that at least one of u and v is 1 or above; and

$$R^{11}O\text{-}(CH_2CH_2O)wH \quad (6)$$

wherein $R^{11}$ represents a linear or branched alkyl or alkenyl group having 10 to 20 carbon atoms; and w is an integer of 0 to 6.

The polyoxyethylene alkyl ether-type nonionic surfactant to be used in the present invention as the component (A) is one represented by the above formula (1). Examples of the linear or branched alkyl or alkenyl group having 10 to 20 carbon atoms represented by $R^1$ in this formula include decyl, undecyl, dodecyl, tridecyl, tetradecyl, pentadecyl, undecenyl, dodecenyl, tridecenyl, tetradecenyl and pentadecenyl groups. In this component (A), it is required that $R^1$ has 10 to 20 carbon atoms and n (i.e., the number of moles of ethylene oxide added) falls within a range of 11 to 30. When these requirements are not satisfied, no sufficient detergency can be achieved.

Among all, it is preferable that $R^1$ has 11 to 15, still preferably 12 to 14, carbon atoms and n is from 14 to 24, since a particularly high detergency can be achieved thereby.

The polyoxyethylene alkyl ether-type nonionic surfactant of the component (A), which serves as the main base of the coloring shampoo composition of the present invention, is contained in an amount of from 7 to 50 % (by weight, the same will apply hereinafter), preferably from 7 to 30 % and still preferably from 9 to 20 %, based on the total composition. When the content thereof is smaller than 7 %, no sufficient detergency can be achieved. On the other hand, it is not desirable that the content exceeds 50 %, since the detergent becomes highly viscous and thus other components can be hardly blended therewith.

As the basic dye to be used as the component (B) in the present invention, any known one may be used without restriction. Either one basic dye or a combination of two or more thereof may be appropriately selected depending on the aimed color tone. Preferable examples thereof include basic dyes Brown No. 17, Brown No. 16, Red No. 76, Yellow No. 57, Blue No. 99, Purple No. 1 and Purple No. 3. In addition, basic dyes preferably employed in the present invention are exemplified by those described in "Grundlagen und rezepturen der Kosmetica", K. Schrader et

al., 2nd ed., Huething Buchverlag Heiderberg (1989).

The basic dye of the component (B) is contained in an amount of from 0.01 to 10 %, preferably from 0.01 to 3 % and still preferably from 0.01 to 1 %, based on the total composition. When the content thereof is smaller than 0.01 %, no sufficient dyeing effect can be obtained. On the other hand, it is not preferable that the content exceeds 10 %, since the composition becomes highly irritative to the skin.

The nonionic surfactant to be used as the component (C) in the present invention is one selected from among alkyldimethylamine oxides represented by the above formula (2), alkyl saccharides represented by the formula (3), fatty acid monoglycerides represented by the formula (4) and alkanolamides represented by the formula (5).

Preferable examples of the alkyldimethylamine oxides represented by the formula (2) are those wherein R2 is an alkyl group having 10 to 16 carbon atoms, still preferably a decyl, lauryl or myristyl group. Preferable examples of the alkyl saccharides represented by the formula (3) are those wherein $R^3$ is an alkyl group having 10 to 14 carbon atoms, still preferably a decyl, dodecyl or tetradecyl group, and $R^4$ is an ethylene or propylene group. The structure of G is determined depending on the monosaccharide, disaccharide or one composed of more saccharide molecules employed as the starting material and particularly preferable examples thereof include glucose, galactose and fructose. s is preferably from 0 to 2, while t is preferably from 1 to 2. Preferable examples of the alkyl saccharides (3) include alkyl polyglucosides wherein s is 0 and G is a residue originating in glucose.

Preferable examples of the fatty acid monoglycerides represented by the formula (4) are those wherein $R^6$ has 8 to 12 carbon atoms. Preferable examples of the alkanolamides represented by the formula (5) are those wherein $R^9$ has 10 to 18 carbon atoms and u and v are each from 0 to 3, and particularly preferable example thereof is isopropanolamide.

Among these substances, the alkyldimethylamine oxides represented by the formula (2) and the fatty acid monoglycerides represented by the formula (4) are preferable from the viewpoint of enhancing the foaming power. It is still preferable to use decanoic acid monoglyceride and lauric acid monoglyceride from among the fatty acid monoglycerides represented by the formula (4).

As the nonionic surfactant of the component (C), use can be made of either one surfactant or a combination of two or more of the same. The content of the nonionic surfactant of the component (C) preferably ranges from 1 to 20 %, still preferably from 3 to 15 %, based on the total composition so as to achieve a sufficient detergency and foaming power.

When the component (C) is employed, the weight ratio of the components (A) and (C) [i.e., (A)/(C)] preferably ranges from 100/1 to 0.4/1, since an improved detergency, little irritation and relieved squeak feel can be achieved thereby. It is still preferable that the weight ratio ranges from 50/1 to 0.5/1, in particular 10/1 to 0.5/1, since the squeak feel can be more relieved and the detergency can be further improved thereby.

In the coloring shampoo composition of the present invention, it is necessary to control the content of the component (A) in such a manner as to regulate the weight ratio of the component (A) to the component (C) [i.e., (A)/(C)] within a range of from 0.5/1 to 10/1. When the weight ratio is excluded from this range, the satisfactory detergency, foaming power and little irritation cannot be achieved at the same time. In order to further enhance the foaming power, it is preferable that the weight ratio falls within a range of from 0.6/1 to 3/1.

Further, the coloring shampoo composition of the present invention may optionally contain other components, if required, so long as the effects of the present invention are not deteriorated thereby. Examples of these components include anionic surfactants, cationic surfactants, nonionic surfactants other than those described above, ampholytic surfactants; components commonly employed in cosmetics, drugs, foods, etc. (for example, anti-dandruff agents, bactericides, anti-inflammatory agents, medicinal components, preservatives); humectants such as propylene glycol, glycerol, diethylene glycol monoethyl ether, sorbitol and panthenol; coloring matters such as dyes other than those described above and pigments; conditioning agents such as perfluoropolyether; pearling agents, chitosan derivatives such as hydroxypropylchitosan; various perfume preparations; and those described in "Encyclopedia of Shampoo Ingredients" (Micelle Press, 1985).

Examples of the above-mentioned ampholytic surfactants include betaine type surfactants such as carbobetaine type, sulfobetaine type and imidazolium betaine type surfactants. Among these substances, preferable ones are carbobetaine type surfactants represented by the following formula (7):

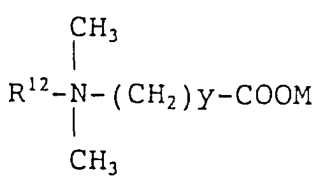

(7)

wherein $R^{12}$ represents a linear or branched alkyl or alkenyl group having 8 to 20 carbon atoms or a group represented by $R^{13}CONH(CH_2)z$- (wherein $R^{13}$ represents a linear or branched alkyl or alkenyl group having 8 to 20 carbon atoms; and z is an integer of from 1 to 5); M represents a hydrogen atom, an alkali metal or triethanolamine; and y is an integer of from 1 to 5; and sulfobetaine type surfactants represented by the following formula (8):

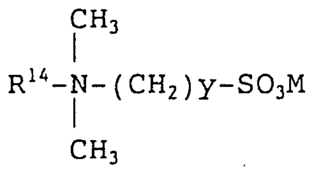

(8)

wherein $R^{14}$ represents a linear or branched alkyl or alkenyl group having 8 to 20 carbon atoms; and M and y are each as defined above.

Furthermore, it is preferable to use carbobetaine type surfactants represented by the formula (7) and, among all, fatty acid amidopropylbetaines wherein $R^{12}$ is $R^{13}CONH(CH_2)z$- are still preferable.

The coloring shampoo composition of the present invention can be produced by mixing the above-mentioned components together by a conventional method.

The coloring shampoo composition of the present invention can exhibit an intense and long-lasting hair-dyeing effect and a good detergency. Moreover, it is little irritative and imparts a good touch to the hair without giving no squeak feel during shampooing.

To further illustrate the present invention in greater detail, and not by way of limitation, the following Examples will be given.

EXAMPLE 1

Coloring shampoos of the compositions as listed in Tables 1 to 3 were produced by a conventional method. Then each product was evaluated in the dyeing characteristics, detergency, foaming power, the touch of the hair during shampooing and skin irritation. The results are given in Tables 1 to 3.

(Evaluation method)

(1) Dyeing characteristics:

0.3 g of each shampoo solution was applied to 1 g of goat hair, foamed for 30 seconds and thoroughly rinsed away with warm water followed by drying. After repeating this procedure thrice, the color of the goat hair was measured with a color difference meter (Model CR200, manufactured by Minolta Camera Co., Ltd.). Then the color difference (dE) from the starting goat hair was determined and the dyeing characteristics were evaluated in accordance with the following criteria. A larger dE means the better dyeing characteristics.

$$dE = [(dL)^2 + (da)^2 + (db)^2]^{1/2}$$

dL: difference in L value (lightness) between the treated hair and the untreated one.
da: difference in a value (reddishness) between the treated hair and the untreated one.
db: difference in b value (yellowness) between the treated hair and the untreated one.
◎: $15 \leq dE$.
O: $10 \leq dE < 15$.
Δ: $5 \leq dE < 10$.
x: $dE < 5$.

(2) Detergency

1 g of each shampoo solution was applied to 20 g (15 cm) of the hair of a healthy Japanese woman. After shampooing for 1 minute and rinsing, the touch of the hair was evaluated by 20 skilled panelists in accordance with the following criteria.

◎: very good (regarded as "good detergency" by more than 90 % of the panelists).

O: good (regarded as "good detergency" by 80 to 90 % of the panelists).
Δ: somewhat poor (regarded as "good detergency" by 70 to 80 % of the panelists).
x: poor (regarded as "good detergency" by less than 70 % of the panelists).

(3) Foaming power

800 ml of each shampoo solution diluted 20-fold (material temperature: 20 °C) was poured into a cylinder and stirred with stirring blades submerged in the aqueous solution for 10 minutes. After allowing to stand for 30 seconds, the foaming power was evaluated in accordance with the following criteria. The stirring blades were rotated at 2,000 rpm and reversed at intervals of 5 seconds.

◎: very good foaming.
O: good foaming.
Δ: foaming but insufficient.
x: scarcely foaming.

(4) Hair touch at shampooing

1 g of each shampoo solution was applied to 20 g (15 cm) of the hair of a healthy woman. After shampooing for 1 minute and rinsing, the touch of the hair was evaluated by 20 skilled panelists in accordance with the following criteria.

◎: very smooth finger passing with no squeak feel.
O: smooth finger passing with a little squeak feel.
Δ: somewhat smooth finger passing with squeak feel.
x: less smooth finger passing with serious squeak feel.

(5) Skin irritation

20 skilled panelists soaked their hands in each shampoo solution for 5 minutes followed by drying. After repeating this procedure 10 times, the skin irritation was evaluated in accordance with the following criteria.

◎: regarded as "little irritative" by more than 90 % of the panelists.
O: regarded as "little irritative" by 80 to 90 % of the the panelists.
Δ: regarded as "little irritative" by 70 to 80 % of the panelists.
x: regarded as "little irritative" by less than 70 % of the panelists.

Table 1

| Component (%) | Invention product | | | | |
|---|---|---|---|---|---|
| | 1 | 2 | 3 | 4 | 5 |
| polyoxyethylene alkyl ether($C_{12}$, EO=20) | 15 | 15 | 15 | 15 | 15 |
| polyoxyethylene alkyl ether($C_8$, EO=20) | | | | | |
| polyoxyethylene alkyl ether($C_{12}$, EO=40) | | | | | |
| polyoxyethylene nonylphenyl ether (EO=20) | | | | | |
| lauryl ether sulfate Na salt (EO=3) | | | | | |
| lauryldimethylamine oxide | | | 2 | 2 | |
| decanoic acid monoglyceride | | | | | 2 |
| decyl glucoside [in formula (3), $R^3=C_{10}H_{21}$, s=0, G=glucose residue, t=1.2] | | 2 | | | |
| lauroyldiethanolamide | | | 2 | | |
| oleamide monoisopropanolamide | | | | 2 | |
| polyoxyethylene alkyl ether ($C_{12}$, EO=4) | | | | | 3 |
| basic dye Red No. 76 | 0.01 | 0.01 | 0.01 | 0.01 | 0.01 |
| acidic dye Orange No. 205 | | | | | |

Table 1 (cont'd)

| | Invention product | | | | |
|---|---|---|---|---|---|
| | 1 | 2 | 3 | 4 | 5 |
| water | the balance | ← | ← | ← | ← |
| dyeing characteristics | ◎ | ◎ | ◎ | ◎ | ◎ |
| detergency | ◎ | ◎ | ◎ | ◎ | ◎ |
| foaming power | O | ◎ | ◎ | ◎ | ◎ |
| hair-touch at shampooing | ◎ | O | ◎ | ◎ | ◎ |
| skin irritation | ◎ | ◎ | ◎ | ◎ | ◎ |

Table 2

| Component (%) | Comparative product 1 | 2 | 3 | 4 | 5 | 6 |
|---|---|---|---|---|---|---|
| polyoxyethylene alkyl ether($C_{12}$, EO=20) | | | | | | |
| polyoxyethylene alkyl ether($C_8$, EO=20) | 15 | | | | 15 | |
| polyoxyethylene alkyl ether($C_{12}$, EO=40) | | 15 | | | | 15 |
| polyoxyethylene nonylphenyl ether (EO=20) | | | 15 | | | |
| lauryl ether sulfate Na salt (EO=3) | | | | 15 | | |
| lauryldimethylamine oxide | | | | | | |
| decanoic acid monoglyceride | | | | | | |
| decyl glucoside [in formula (3), $R^3=C_{10}H_{21}$, s=0, G=glucose residue, t=1.2] | | | | | 2 | 2 |
| lauroyldiethanolamide | | | | | | |
| basic dye Red No. 76 | 0.01 | 0.01 | 0.01 | 0.01 | 0.01 | 0.01 |
| acidic dye Orange No. 205 | | | | | | |
| water | the balance | ← | ← | ← | ← | ← |

Table 2 (cont'd)

| | Comparative product | | | | | |
|---|---|---|---|---|---|---|
| | 1 | 2 | 3 | 4 | 5 | 6 |
| dyeing characteristics | ◎ | ◎ | ◎ | x | ◎ | ◎ |
| detergency | △ | △ | O | ◎ | △ | △ |
| foaming power | x | △ | △ | ◎ | x | △ |
| hair-touch at shampooing | △ | △ | △ | △ | △ | △ |
| skin irritation | O | O | △ | x | O | O |

Table 3

| Component (%) | Comparative product 7 | 8 | 9 | 10 | 11 |
| --- | --- | --- | --- | --- | --- |
| polyoxyethylene alkyl ether($C_{12}$, EO=20) | | | | 3 | 3 |
| polyoxyethylene alkyl ether($C_8$, EO=20) | | | | | |
| polyoxyethylene alkyl ether($C_{12}$, EO=40) | | | | | |
| polyoxyethylene nonylphenyl ether (EO=20) | 15 | | | | |
| lauryl ether sulfate Na salt (EO=3) | | 15 | | | |
| lauryldimethylamine oxide | | | | | |
| decanoic acid monoglyceride | | | | | |
| decyl glucoside [in formula (3), $R^3=C_{10}H_{21}$, s=0, G=glucose residue, t=1.2] | 2 | 2 | | 10 | |
| lauroyldiethanolamide | | | | | |
| basic dye Red No. 76 | 0.01 | 0.01 | 0.01 | 0.01 | 0.01 |
| acidic dye Orange No. 205 | | | | | |
| water | the balance | ← | ← | ← | ← |

Table 3 (cont'd)

| | Comparative product | | | | |
|---|---|---|---|---|---|
| | 7 | 8 | 9 | 10 | 11 |
| dyeing characteristics | ◎ | × | ○ | ◎ | △ |
| detergency | ○ | ◎ | × | ○ | ○ |
| foaming power | △ | ◎ | × | ○ | ○ |
| hair-touch at shampooing | △ | △ | ○ | × | ◎ |
| skin irritation | △ | × | ○ | ○ | ○ |

EXAMPLE 2

A coloring shampoo of the following composition was produced by a conventional method.

This shampoo was excellent in the foaming power and detergency and exhibited an intense and long-lasting effect of dyeing red. Moreover, it was less irritative and imparted a good touch to the hair while giving no squeak feel during shampooing.

Table 6

| (Component) | (%) |
|---|---|
| polyoxyethylene (15) lauryl ether | 10.00 |
| laurylhydroxysulfobetaine | 1.00 |
| alkylglucoside[1)] | 2.00 |
| decanoic acid monoglyceride | 3.00 |
| cationized cellulose[2)] | 0.30 |
| hydroxyethylcellulose[3)] | 0.10 |
| silicone emulsion[4)] | 1.00 |
| basic dye Red No. 76 | 0.08 |
| basic dye Blue No. 99 | 0.01 |
| basic dye Yellow No. 57 | 0.01 |
| pH regulator | q.s. |
| perfume | q.s. |
| water | the balance |
| | 100.0 |

1) In formula (3), $R^3 = C_{10}H_{21}$, s = 0, G = glucose residue, t = 1.2.
2) JR400 (manufactured by UCC).
3) HEC-SE850 (manufactured by Shin-Etsu Chemical Co., Ltd.).
4) SM-8702C (manufactured by Toray-Dow Corning).

EXAMPLE 3

A coloring shampoo of the following composition was produced by a conventional method.
This shampoo was excellent in the foaming power and detergency and exhibited an intense and long-lasting effect of dyeing red. Moreover, it was less irritative and imparted a good touch to the hair while giving no squeak feel during shampooing.

Table 7

| (Component) | (%) |
|---|---|
| polyoxyethylene (15) lauryl ether | 10.00 |
| polyoxyethylene (3) lauryl ether | 3.00 |
| lauryldimethylamineoxide | 1.00 |
| alkylglucoside[1)] | 5.00 |
| decanoic acid monoglyceride | 1.00 |
| oleic acid monoisopropanolamide | 3.00 |
| cationized cellulose[2)] | 0.30 |
| hydroxyethylcellulose[3)] | 0.10 |
| silicone emulsion[4)] | 1.00 |
| basic dye Red No. 76 | 0.08 |
| basic dye Blue No. 99 | 0.01 |
| basic dye Yellow No. 57 | 0.01 |
| pH regulator | q.s. |
| perfume | q.s. |

2) JR400 (manufactured by UCC).
2) JR400 (manufactured by UCC).
3) HEC-SE850 (manufactured by Shin-Etsu Chemical Co., Ltd.).
4) SM-8702C (manufactured by Toray-Dow Corning).

Table 7 (continued)

| (Component) | (%) |
|---|---|
| water | the balance |
| | 100.0 |

## Claims

1. A coloring shampoo composition which comprises the components (A) and (B) as defined below:

   (A) 7 to 50 % by weight of a polyoxyethylene alkyl ether-type nonionic surfactant represented by the following formula (1):

   $$R^1O\text{-}(CH_2CH_2O)n\text{-}H \qquad (1)$$

   wherein $R^1$ represents a linear or branched alkyl or alkenyl group having 10 to 20 carbon atoms; and n is a number of from 11 to 30; and
   (B) 0.01 to 10 % by weight of a basic dye.

2. A coloring shampoo composition as claimed in Claim 1 which further contains:
   (C) 0.1 to 30 % by weight of at least one nonionic surfactant selected from among those represented by the following formulae (2) to (6) :

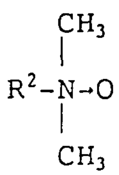

(2)

wherein $R^2$ represents a linear or branched alkyl or alkenyl group having 8 to 18 carbon atoms;

$$R^3\text{-}(OR^4)s\text{-}Gt \qquad (3)$$

wherein $R^3$ represents a linear or branched alkyl or alkenyl group having 6 to 18 carbon atoms; $R^4$ represents an alkylene group having 2 to 4 carbon atoms; G represents a group originating in a reducing sugar having 5 or 6 carbon atoms; s is a number of from 0 to 10; and t is a number of from 1 to 10;

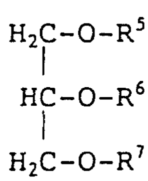

(4)

wherein either one of $R^5$, $R^6$ and $R^7$ represents $O=CR^8$ (wherein $R^8$ represents a linear or branched alkyl or alkenyl group having 6 to 18 carbon atoms), while others represent each a hydrogen atom;

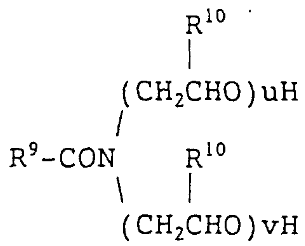 (5)

wherein $R^9$ represents a linear or branched alkyl or alkenyl group having 8 to 20 carbon atoms; $R^{10}$ is a hydrogen atom or methyl group; and u and v are each an integer of 0 to 10, provided that at least one of u and v is 1 or above; and

$$R^{11}O\text{-}(CH_2CH_2O)wH \quad (6)$$

wherein $R^{11}$ represents a linear or branched alkyl or alkenyl group having 10 to 20 carbon atoms; and w is an integer of 0 to 6.

**3.** A coloring shampoo composition as claimed in Claim 2, wherein the weight ratio of the component (A) to the component (C) [(A)/(C)] ranges from 100/1 to 0.4/1.

**4.** A coloring shampoo composition as claimed in Claim 2, wherein the weight ratio of the component (A) to the component (C) [(A)/(C)] ranges from 50/1 to 0.5/1.

**5.** A coloring shampoo composition as claimed in Claim 2, wherein the weight ratio of (A)/(C) ranges from 10/1 to 0.5/1.

**6.** A coloring shampoo composition as claimed in Claim 2, wherein the weight ratio of (A)/(C) ranges from 3/1 to 0.6/1.

**7.** A hair-dyeing method which comprises shampooing the hair with a coloring shampoo composition as claimed in any of Claims 1 to 6.

**8.** Use of a coloring shampoo composition as claimed in any of Claims 1 to 6 as a hair-coloring shampoo.

**Patentansprüche**

**1.** Färbeshampoo-Zusammensetzung, die die unten definierten Komponenten (A) und (B) umfasst:

(A) 7 bis 50 Gew.% eines nicht-ionischen Tensids vom Polyoxyethylenalkylether-Typ, dargestellt durch die folgende Formel (1) :

$$R^1O\text{-}(CH_2CH_2O)_n\text{-}H \quad (1)$$

wobei $R^1$ eine geradkettige oder verzweigte Alkyl- oder Alkenylgruppe mit 10 bis 20 Kohlenstoffatomen darstellt; und n eine Zahl von 11 bis 30 ist; und

(B) 0,01 bis 10 Gew.% eines basischen Farbstoffs.

**2.** Färbeshampoo-Zusammensetzung nach Anspruch 1, die weiterhin enthält:
(C) 0,1 bis 30 Gew.% mindestens eines nicht-ionischen Tensids, ausgewählt aus solchen, die durch die folgenden Formeln (2) bis (6) dargestellt sind:

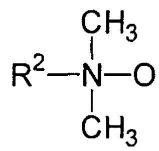

(2)

wobei $R^2$ eine geradkettige oder verzweigte Alkyl- oder Alkenylgruppe mit 8 bis 18 Kohlenstoffatomen darstellt;

$$R^3—(OR^4)_s—G_t \quad (3)$$

wobei $R^3$ eine geradkettige oder verzweigte Alkyl- oder Alkenylgruppe mit 6 bis 18 Kohlenstoffatomen darstellt; $R^4$ eine Alkylengruppe mit 2 bis 4 Kohlenstoffatomen darstellt; G eine Gruppe darstellt, die von einem reduzierenden Zucker mit 5 oder 6 Kohlenstoffatomen abgeleitet ist; s eine Zahl von 0 bis 10 ist; und t eine Zahl von 1 bis 10 ist;

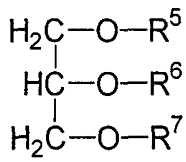

(4)

wobei ein Vertreter von $R^5$, $R^6$ und $R^7$ $O{=}CR^8$ darstellt, wobei $R^8$ eine geradkettige oder verzweigte Alkyl- oder Alkenylgruppe mit 6 bis 18 Kohlenstoffatomen darstellt, während die anderen jeweils ein Wasserstoffatom darstellen;

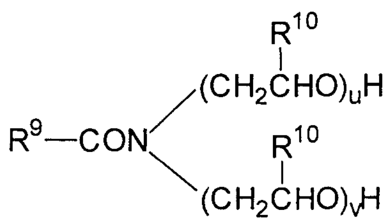

(5)

wobei $R^9$ eine geradkettige oder verzweigte Alkyl- oder Alkenylgruppe mit 8 bis 20 Kohlenstoffatomen darstellt; $R^{10}$ ein Wasserstoffatom oder eine Methylgruppe ist; und u und v jeweils ganze Zahlen von 0 bis 10 sind, unter der Massgabe, dass u und/oder v 1 oder mehr beträgt; und

$$R^{11}O\text{-}(CH_2CH_2O)_wH \quad (6)$$

wobei $R^{11}$ eine geradkettige oder verzweigte Alkyl- oder Alkenylgruppe mit 10 bis 20 Kohlenstoffatomen darstellt; und w eine ganze Zahl von 0 bis 6 ist.

**3.** Färbeshampoo-Zusammensetzung nach Anspruch 2, wobei das Gewichtsverhältnis der Komponente (A) zu der Komponente (C) [(A)/(C)] im Bereich von 100:1 bis 0,4:1 liegt.

**4.** Färbeshampoo-Zusammensetzung nach Anspruch 2, wobei das Gewichtsverhältnis der Komponente (A) zu der Komponente (C) [(A)/(C)] im Bereich von 50:1 bis 0,5:1 liegt.

**5.** Färbeshampoo-Zusammensetzung nach Anspruch 2, wobei das Gewichtsverhältnis von [(A)/(C)] im Bereich von 10:1 bis 0,5:1 liegt.

**6.** Färbeshampoo-Zusammensetzung nach Anspruch 2, wobei das Gewichtsverhältnis von [(A)/(C)] im Bereich von

3:1 bis 0,6:1 liegt.

**7.** Haarfärbeverfahren, das eine Shampoobehandlung des Haares mit einer Färbeshampoo-Zusammensetzung nach einem der Ansprüche 1 bis 6 umfasst.

**8.** Verwendung einer Färbeshampoo-Zusammensetzung nach einem der Ansprüche 1 bis 6 als ein Haarfärbeshampoo.

## Revendications

**1.** Composition de shampooing colorant comprenant les constituants (A) et (B) définis ci-dessous:

(A) 7 à 50 % en masse d'un agent tensioactif non ionique de type éther de polyoxyéthylène et d'alkyle représenté par la formule (I) suivante:

$$R^1O\text{-}(CH_2CH_2O)_n\text{-}H \qquad (1)$$

dans laquelle $R^1$ représente un groupe alkyle ou alcényle linéaire ou ramifié de 10 à 20 atomes de carbone; et n est un nombre de 11 à 30; et
(B) 0,01 à 10 % en masse d'un colorant basique.

**2.** Composition de shampooing colorant selon la revendication 1, qui contient en outre:
(C) 0,1 à 30 % en masse d'au moins un agent tensioactif non ionique choisi parmi ceux représentés par les formules générales (2) à (6) suivantes:

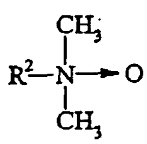

(2)

dans laquelle $R^2$ représente un groupe alkyle ou alcényle linéaire ou ramifié de 8 à 18 atomes de carbone;

$$R^3\text{-}(OR^4)_s\text{-}G_t \qquad (3)$$

dans laquelle $R^3$ représente un groupe alkyle ou alcényle linéaire ou ramifié de 6 à 18 atomes de carbone; $R^4$ représente un groupe alkylène de 2 à 4 atomes de carbone; G représente un groupe dérivé d'un sucre réducteur ayant 5 ou 6 atomes de carbone; s est un nombre de 0 à 10; et t est un nombre de 1 à 10;

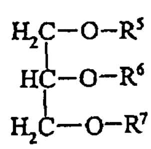

(4)

dans laquelle l'un ou l'autre des $R^5$, $R^6$ et $R^7$ représente un groupe $O{=}CR^8$ (dans lequel $R^8$ représente un groupe alkyle ou alcényle linéaire ou ramifié de 6 à 18 atomes de carbone) et les autres représentent chacun un atome d'hydrogène;

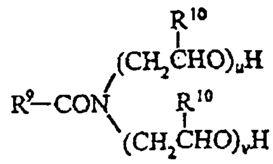

(5)

dans laquelle $R^9$ représente un groupe alkyle ou alcényle linéaire ou ramifié de 8 à 20 atomes de carbone; $R^{10}$ est un atome d'hydrogène ou un groupe méthyle; et u et v sont chacun un entier de 0 à 10, à condition qu'au moins l'un des u et v soit égal ou supérieur à 1 ; et

$$R^{11}O\text{-}(CH_2CH_2O)_wH \qquad (6)$$

dans laquelle $R^{11}$ représente un groupe alkyle ou alcényle linéaire ou ramifié de 10 à 20 atomes de carbone; et w est un entier de 0 à 6.

**3.** Composition de shampooing colorant selon la revendication 2, dans laquelle le rapport en masse du constituant (A) au constituant (C) [(A)/(C)] est compris entra 100/1 et 0,4/1.

**4.** Composition de shampooing colorant selon la revendication 2, dans laquelle le rapport en masse du constituant (A) au constituant (C) [(A)/(C)] est compris entre 50/1 et 0,5/1.

**5.** Composition de shampooing colorant selon la revendication 2, dans laquelle le rapport en masse (A)/(C) est compris entre 10/1 et 0,5/1.

**6.** Composition de shampooing colorant selon la revendication 2, dans laquelle le rapport en masse (A)/(C) est compris entre 3/1 et 0,6/1.

**7.** Procédé de coloration des cheveux dans lequel on shampouine les cheveux avec une composition de shampooing colorant selon l'une quelconque des revendications 1 à 6.

**8.** Utilisation d'une composition de shampooing colorant selon l'une quelconque des revendications 1 à 6 comme shampooing colorant pour les cheveux.